# EUROPEAN PATENT APPLICATION

(11) **EP 4 831 619 A1**
(43) Date of publication of application: **28.10.2026**
(21) Application number: 26169530.8
(22) Date of filing: 31.03.2026
(51) Int. Cl.: G01N 21/62

(54) **BIOSENSOR**

(30) Priority: 23.04.2025 US 202563793539 P; 17.11.2025 US 202519392108
(71) Applicant: Samsung Electronics Co., Ltd., Suwon-si, Gyeonggi-do 16677 (KR)
(72) Inventor: KUMAR, Shailabh, San Jose, CA, 95134 (US); SIDDIQUE, Radwanul Hasan, San Jose, CA, 95134 (US)
(74) Representative: Kuhnen & Wacker Patent- und Rechtsanwaltsbüro PartG mbB

(57) **Abstract**

A biosensor (105). In some embodiments, a system (105, 110) includes: a biosensor (105), including: an infrared transmitter; and an infrared receiver, the infrared transmitter including a light source and a first metasurface, the infrared transmitter being configured to transmit light, from the light source, through the first metasurface, the infrared receiver including a detector and a second metasurface, the detector being configured to receive light which enters the infrared receiver through the second metasurface.

## Description

### FIELD

One or more aspects of embodiments according to the present disclosure relate to optical sensing systems, and more particularly to an optical biosensor.

### BACKGROUND

Light, e.g., thermal infrared light, may propagate within human tissue, and various characteristics of the tissue may affect the interactions between the light and the tissue.

It is with respect to this general technical environment that aspects of the present disclosure are related.

The above information disclosed in this Background section is only for enhancement of understanding of the background and therefore the information discussed in this Background section does not necessarily constitute prior art.

### SUMMARY

According to an embodiment of the present disclosure, there is provided a biosensor, including: an infrared transmitter; and an infrared receiver, the infrared transmitter including a light source and a first metasurface, the infrared transmitter being configured to transmit light, from the light source, through the first metasurface, the infrared receiver including a detector and a second metasurface, the detector being configured to receive light which enters the infrared receiver through the second metasurface.

In some embodiments, the first metasurface functions as a polarizer.

In some embodiments, the first metasurface further functions as a lens.

In some embodiments, the second metasurface functions as a polarizer.

In some embodiments, the first metasurface is oriented so that the infrared transmitter emits vertically polarized light.

In some embodiments, the second metasurface is oriented so as to reject light transmitted by the infrared transmitter and received at the receiver via a single specular reflection.

In some embodiments, the first metasurface is oriented so that the infrared transmitter emits vertically polarized light.

In some embodiments, the second metasurface further functions as a lens.

In some embodiments, the second metasurface further functions as a disperser.

In some embodiments, the second metasurface further functions as a lens.

In some embodiments, the first metasurface functions as a lens.

In some embodiments: the light source includes a first pixel and a second pixel; and the first metasurface includes: a first pixel aligned with the first pixel of the light source, and a second pixel aligned with the second pixel of the light source.

In some embodiments, the biosensor further includes a temperature sensor configured to measure ambient temperature.

In some embodiments, the light source includes an element selected from the group consisting of broadband thermal sources, quantum cascade lasers, optical parametric oscillators, and super continuum lasers.

In some embodiments, the detector includes an element selected from the group consisting of thermopile detectors, pyroelectric detectors, bolometers, photonic detectors, and focal plane arrays.

According to an embodiment of the present disclosure, there is provided an apparatus, including: an electronic device, including the biosensor.

According to an embodiment of the present disclosure, there is provided a biosensor, including: a transmitter; and a receiver, the transmitter including a light source and a first metasurface, the transmitter being configured to transmit light, from the light source, through the first metasurface, the receiver including a detector and a second metasurface, the detector being configured to receive light which enters the receiver through the second metasurface, the second metasurface functioning as a lens.

In some embodiments, the first metasurface functions as a polarizer.

In some embodiments, the second metasurface functions as a polarizer.

According to an embodiment of the present disclosure, there is provided a biosensor, including: a transmitter; and a receiver, the transmitter including a light source and a first metasurface, the transmitter being configured to transmit light, from the light source, through the first metasurface, the receiver including a detector and a second metasurface, the detector being configured to receive light which enters the receiver through the second metasurface, the second metasurface functioning as a lens and as a polarizer.

### BRIEF DESCRIPTION OF THE DRAWINGS

These and other features and advantages of the present disclosure will be appreciated and understood with reference to the specification, claims, and appended drawings wherein:
FIG. 1 is a system-level diagram of a computing device and a biosensor, according to an embodiment of the present disclosure;
FIG. 2A is a block diagram of a biosensor, according to an embodiment of the present disclosure;
FIG. 2B is a block diagram of a system on a chip including a biosensor, according to an embodiment of the present disclosure;
FIG. 3A is a block diagram of a biosensor in use, according to an embodiment of the present disclosure;
FIG. 3B is a block diagram of a biosensor in use, according to an embodiment of the present disclosure; and
FIG. 4 is a schematic view of a plurality of electronic devices, according to an embodiment of the present disclosure.

### DETAILED DESCRIPTION

The detailed description set forth below in connection with the appended drawings is intended as a description of exemplary embodiments of a biosensor provided in accordance with the present disclosure and is not intended to represent the only forms in which the present disclosure may be constructed or utilized. The description sets forth the features of the present disclosure in connection with the illustrated embodiments. It is to be understood, however, that the same or equivalent functions and structures may be accomplished by different embodiments that are also intended to be encompassed within the scope of the disclosure. As denoted elsewhere herein, like element numbers are intended to indicate like elements or features.

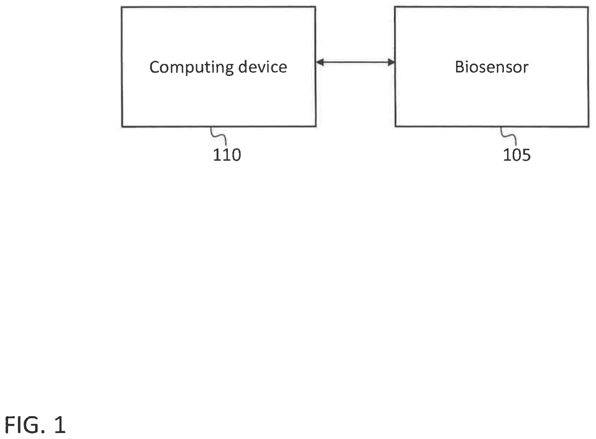

FIG. 1 is a top-level drawing of a system including a biosensor 105 and a computing device 110. The biosensor 105 may be an optical biosensor including an optical source (or "light source") that illuminates the tissue of a subject with light (e.g., visible light, ultraviolet light, or infrared light) and an optical detector that receives a portion of the light from the light source, after this light has interacted with the tissue of the subject. The biosensor 105 may measure the effect, on the light, of interaction between the illuminating (or "probe") light and the tissue of the subject. The biosensor 105 may then infer an aspect of the state of the tissue from the measured effect. For example, if the effect of the tissue is to absorb light strongly at a first wavelength, and if the first wavelength is the wavelength of an absorption peak of a biomolecule of interest, then the biosensor 105 may infer that the biomolecule of interest is present in the tissue. In some embodiments, the biosensor 105 may infer the concentration of the biomolecule of interest, in addition to inferring the presence of the biomolecule of interest, based on the interaction of the probe light with the tissue of the subject.

In some embodiments, the biosensor 105 may infer the state of a biomolecule of interest, based on the interaction of the probe light with the tissue of the subject. For example, the shape of the oxygen-hydrogen (OH) stretching band in water may change with temperature, and this change in shape may be detected, by the biosensor 105, as a change in the interaction of the probe light with the tissue of the subject. As such, in some embodiments, the biosensor 105 may infer the temperature of the tissue of the subject based on the interaction of the probe light with the tissue of the subject.

In some embodiments, the biosensor 105 may detect light other than that produced by the light source of the biosensor 105. For example, the biosensor 105 may detect both ambient light directly received by the biosensor 105, and ambient light that has interacted with the tissue of the subject. In another embodiment, the biosensor 105 may detect blackbody radiation emitted by the tissue of the subject, and infer the temperature, or the composition of the tissue of the subject, based on the light emitted by (e.g., based on the spectrum of the light emitted by) the tissue of the subject. As used herein, the "subject" means a person (e.g., a patient) whose tissue is to be analyzed by the biosensor 105, and the "user" is the person operating the biosensor 105. As such, the user may be the subject, or, for example, a clinician.

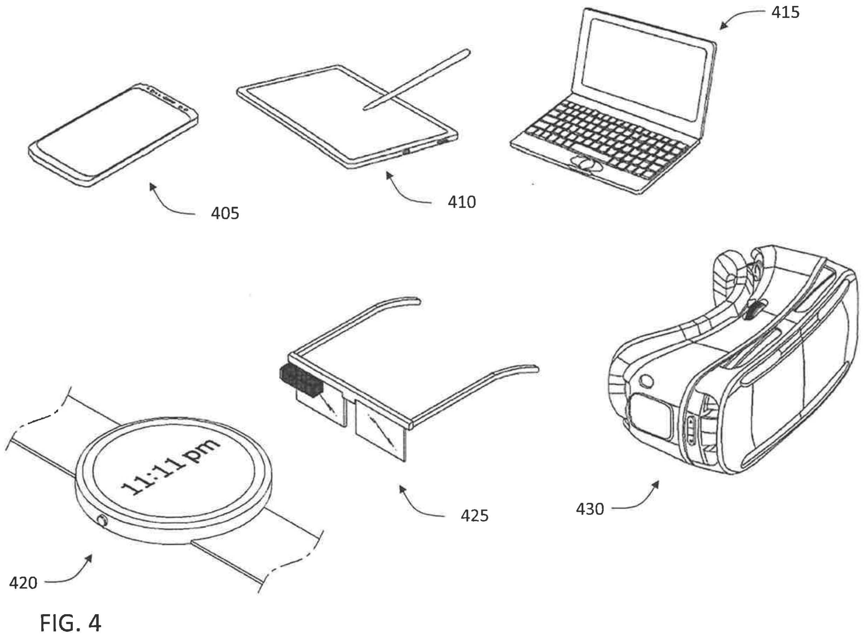

The biosensor 105 may be configured to be worn by the subject, e.g., secured by a strap to the wrist of the subject, or to the chest of the subject. In some embodiments, the biosensor 105 is worn on the neck, on the head, on the leg (e.g., on the ankle), on a finger, on the face (as a pair of glasses or goggles (FIG. 4)) or in an ear. The biosensor 105 may be constructed with a form factor making it suitable to be worn in this manner. For example, a biosensor 105 configured to be worn in the ear of the subject may have the form factor of an ear bud, and a biosensor 105 configured to be worn on the wrist of the subject may have the form factor of a wristwatch.

In some embodiments, the biosensor 105 is embedded in a card, which may be, e.g., square or rectangular, with dimensions such that the subject's finger or thumb may be placed on the biosensor 105 embedded in the card. Such a card may have dimensions of at least 1 centimeter by 1 centimeter, and at most 10 centimeters by 10 centimeters, and a thickness of at most 10 mm. Similarly, an electronic device such as a laptop, a mobile telephone, or a tablet computer may incorporate the biosensor 105. In such an embodiment, to use the biosensor 105, the subject may place her or his thumb or finger on the surface of the electronic device at a point at which the biosensor 105 is located within the electronic device (e.g., behind a window in the housing of the electronic device).

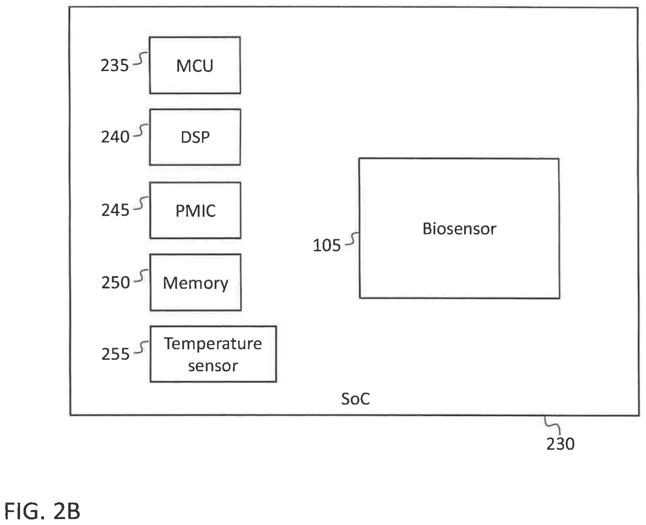

In some embodiments, the computing device 110 is a separate device from the biosensor 105, as illustrated in FIG. 1. The computing device 110 may be, for example, a mobile telephone or a portable computer such as a tablet computer or a laptop computer. The computing device 110 may be connected to the biosensor 105, as shown in FIG. 1, and it may be configured to send instructions or data to the biosensor 105 or to receive instructions or data from the biosensor 105. In some embodiments, the computing device 110 is integrated into (e.g., incorporated into the housing of) the biosensor 105 (as discussed in further detail below, in the context of FIG. 2B) or the biosensor 105 is integrated into (e.g., incorporated into the housing of) the computing device 110 (as discussed in further detail below, in the context of FIG. 4, in which the computing device 110 is, or is part of, an electronic device).

The computing device 110 may perform various functions in the system of FIG. 1, including (i) operating as a user input device, (ii) operating as an output device, e.g., as a display for showing measurement results to the user, (iii) operating as a data storage and processing device, and (iv) providing connectivity to other resources, such as other data storage or processing devices (e.g., cloud data storage or cloud processing).

For example, in operation, the computing device 110 may receive an instruction to perform a measurement, from the user (who may, for example, tap a suitably labeled button on a graphical user interface displayed by the computing device 110). The computing device 110 may then, in response to the instruction received from the user, instruct the biosensor 105 to perform the measurement. The biosensor may perform the measurement and send, e.g., raw or processed data to the computing device 110, which may perform any needed further data processing and display the results to the user.

The computing device 110 may further store the raw data or the processed data from the measurement in the cloud (e.g., in a server connected to the internet), e.g., in a database in which the data is associated with an account of the user or the subject. The computing device 110 may also store metadata along with the raw or processed measurement data; such metadata may include, for example, the date and time at which the measurement was performed.

The connection between the biosensor 105 and the computing device 110 may be a wired connection or a wireless connection. For example, the biosensor 105 may be connected to the computing device 110 by a Universal Serial Bus (USB) cable, which may allow bidirectional transfer of data and instructions, and which may also allow the computing device 110 to supply power to the biosensor 105. In other embodiments, as mentioned above, the connection between the biosensor 105 and the computing device 110 may be a wireless connection. Such a connection may be a WiFi connection or a Bluetooth connection, or a near-field communication (NFC) connection. A wired connection may have the advantage of greater data security and of the ability to transfer power, whereas a wireless connection may have the advantage of greater convenience.

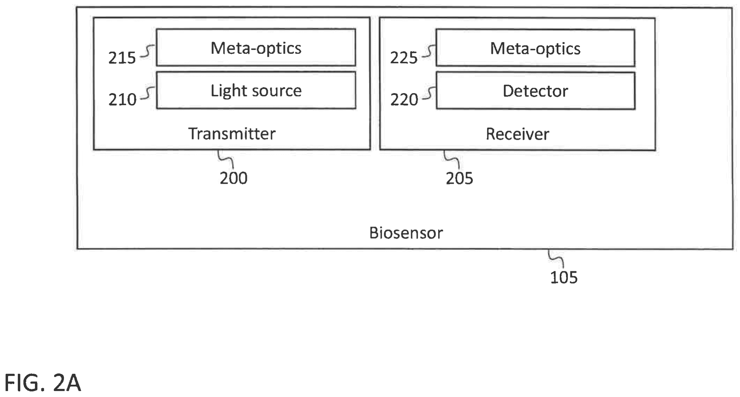

FIG. 2A shows a biosensor 105, in some embodiments. The biosensor 105 includes a transmitter 200 for transmitting light (e.g., the probe light) into the tissue of the subject and a receiver 205 for receiving a portion of the probe light after it interacts with the tissue of the subject. The transmitter 200 may include (as mentioned above) a light source 210 for generating the probe light. The transmitter 200 may also include, as shown, a first set of (one or more) meta-optics 215, through which the probe light may be transmitted from the transmitter 200. As used herein, a "set of meta-optics" means one or more optical elements including at least one metasurface. In some embodiments, the transmitter 200 is a thermal infrared transmitter, e.g., it is capable of transmitting a wavelength that is in the thermal infrared region of the electromagnetic spectrum (discussed in further detail below). In some embodiments, the transmitter 200 is capable of transmitting a plurality of wavelengths extending across at least 80% of the thermal infrared region of the electromagnetic spectrum. The light source may include an element (e.g., a light-emitting element) selected from the group consisting of broadband thermal sources, quantum cascade lasers, optical parametric oscillators, and super continuum lasers. The light source may include (e.g., consist of) a single emitter of light (which may be referred to as a "bulk" emitter) or it may be pixelated, e.g., it may include an array of independently controllable emitters.

In some embodiments, the biosensor 105 uses light that is in a thermal infrared region of the electromagnetic spectrum. The "thermal infrared" region of the electromagnetic spectrum may extend, for example, from 1 micrometer (micron) to 12 micrometers. The thermal infrared region of the electromagnetic spectrum may be a portion of the infrared spectrum (the "infrared" region of the electromagnetic spectrum may extend, for example, from 780 nm to 1 mm). The thermal infrared region of the electromagnetic spectrum may be well suited for performing measurements of the kind for which the biosensor 105 may be used because (i) absorption peaks of biomolecules of interest may be within the thermal infrared region of the electromagnetic spectrum and because (ii) the peak of the blackbody radiation spectrum for an object at human body temperature may be within the thermal infrared region of the electromagnetic spectrum. The detector may include an element (e.g., a light-sensitive element) selected from the group consisting of thermopile detectors, pyroelectric detectors, bolometers, photonic detectors, and focal plane arrays. The detector may include (e.g., consist of) a single detector element (which may be referred to as a "bulk" detector) or it may be pixelated, e.g., it may include an array of independently readable detectors.

As discussed in further detail below, a "metasurface" may be or include a surface that has a plurality of features (e.g., pillars) (which may be referred to as "nanostructures") on the surface, each feature having a largest dimension that is at least 1/10 of the minimum wavelength of the region of the electromagnetic spectrum for which the system/the biosensor is designed (e.g., the thermal infrared region of the electromagnetic spectrum, or a range of wavelengths extending from about 1 micron to about 12 microns), and less than 10 times the maximum wavelength of the region of the electromagnetic spectrum for which the system/the biosensor is designed. A metasurface may include multiple layers of nanostructures, which may be separated by substrates or thin films with low loss (e.g., no loss) and low dielectric index within the infrared spectrum or within the thermal infrared spectrum. For example, a metasurface on a first surface of a substrate may include a plurality of layers of nanostructures alternating with thin films. A metasurface may have characteristics that change continuously across the surface (such a metasurface may be referred to as a "bulk" metasurface) or it may have discrete regions between which the characteristics may change discontinuously (such a metasurface may be referred to as a "pixelated" metasurface, and the discrete regions may be referred to as "pixels" of the metasurface).

For example, each pixel of a pixelated metasurface may function as an optical bandpass filter with a different respective center wavelength. Such a pixelated metasurface may be mounted in front of a pixelated broadband light source 210 (in which each pixel emits broadband radiation) or in front of a pixelated broadband detector 220 (in which each pixel is sensitive to a broad range of wavelengths), with the pixels of the pixelated metasurface aligned with the pixels of the pixelated light source 210 or with the pixels of the pixelated detector 220.

The combination of the pixelated broadband light source 210 and the pixelated metasurface may operate as a tunable light source, in which the wavelength at which the tunable light source emits light may be selectable by selecting which pixel is to be turned on (e.g., caused to emit light). For example, the light source may emit light at a first wavelength when a first pixel of the pixelated broadband light source 210 emits broadband light, the first pixel being aligned with a first pixel of the pixelated metasurface, the first pixel of the pixelated metasurface having a passband with a center wavelength equal to the first wavelength. The combination of the pixelated broadband detector 220 and the pixelated metasurface may operate as a wavelength-discriminating detector which may, for example, be able to measure the received spectral irradiance at each of a plurality of wavelengths. For example, a first pixel of the pixelated broadband detector 220 may be aligned with a first pixel of the pixelated metasurface, the first pixel of the pixelated metasurface being an optical bandpass filter with center wavelength equal to a first wavelength; as a result of this the first pixel of the pixelated broadband detector 220 may primarily detect light at the first wavelength. Similarly, a second pixel of the pixelated broadband detector 220 may be aligned with a second pixel of the pixelated metasurface, the second pixel of the pixelated metasurface being an optical bandpass filter with center wavelength equal to a second wavelength (different from the first wavelength); as a result of this the second pixel of the pixelated broadband detector 220 may primarily detect light at the second wavelength.

The receiver 205 may include a detector 220 and a second set of meta-optics 225. The receiver 205 may be a thermal infrared receiver, e.g., it may be capable of receiving a wavelength within the thermal infrared region of the electromagnetic spectrum. In some embodiments, the receiver 205 may be capable of receiving any wavelength within a portion of the thermal infrared region of the electromagnetic spectrum that extends across at least 80% of the thermal infrared region of the electromagnetic spectrum. In some embodiments one or both of the first set of meta-optics 215 and the second set of meta-optics is absent.

In some embodiments, the biosensor 105 is part of a system on a chip (SoC) 230, as shown in FIG. 2B. The system on a chip 230, which may be a silicon integrated circuit, may include the biosensor 105 or one or more components of the biosensor 105, and other circuits, such as a microcontroller unit (MCU) 235, a digital signal processor (DSP) 240, a power management integrated circuit (PMIC) 245, a memory 250, and a temperature sensor 255, which may be used to measure the ambient temperature to calibrate and remove ambient temperature fluctuations. The elements illustrated in FIG. 2B may be connected to one or more data or control busses, which may operate, for example, under the management of the microcontroller unit 235. In such an embodiment, the microcontroller unit 235 may control the light source 210 (e.g., control the power supplied to the light source 210) and control the detector 220 (e.g., control the acquisition of measurements by the detector 220). The microcontroller unit 235 may further coordinate the transfer of data from the detector 220 to the digital signal processor 240, which may perform analysis of the data generated by the detector 220, to infer, for example, the presence or concentration of a biomolecule of interest within the tissue of the subject. Data transfers between the detector 220 and the digital signal processor 240 may use the memory 250 for buffering, and the power management integrated circuit may provide power (e.g., at respective operating voltages) to each of the other elements of the system. In some embodiments, the system includes a neural processing unit, which may host a neural network used to analyze the data generated by the biosensor 105.

The first set of meta-optics 215 may (i) modify the convergence of the light produced by the light source, (ii) modify the polarization of the light produced by the light source, or (iii) modify the dispersion of the light produced by the light source.

As used herein, the "convergence" of light means the extent to which rays of the light converge or diverge. As such, a converging lens (e.g., a positive lens) may modify the convergence of collimated light by changing it from being collimated to being converging, e.g., by causing the light to focus at a point. Similarly, a diverging lens (e.g., a negative lens) may cause collimated light to diverge, thereby changing the convergence of the light.

As used herein, "modifying the polarization state" of light means changing the polarization state of the light. As used herein, the "dispersion" of light means the extent to which different wavelength components of the light propagate in different directions. For example, a prism or diffraction grating, illuminated by collimated broadband light (in which each wavelength component is propagating in the same direction) may cause different wavelength components of the light to propagate in different directions (e.g., may disperse the light), thereby modifying the dispersion of the light. As used herein, a metasurface (e.g., within the first set of meta-optics 215 or within the second set of meta-optics 225) that disperses light may be referred to as a "disperser".

Similarly, the second set of meta-optics 225 may (i) modify the convergence of the light received by the receiver 205, (ii) modify the polarization of the light received by the receiver 205, or (iii) modify the dispersion of the light received by the receiver 205.

The first set of meta-optics 215 and the second set of meta-optics 225 may affect the operation of the biosensor 105 in various ways. For example, in an embodiment in which the light source 210 emits diverging light, the first set of meta-optics 215 may function as a positive (converging) lens, to collimate the light emitted by the light source or to focus the light from the light source at a point that is at a desired depth within the tissue of the subject. Such collimating or focusing may increase the fraction of the light emitted by the light source 210 that interacts with the tissue of the subject and is then received by the receiver 205. Similarly, if the light-sensitive area of the detector 220 is smaller than the receiving aperture of the receiver 205, the second set of meta-optics 225 may function as a converging lens, to focus or concentrate the light that is received through the receiving aperture of the receiver 205 onto the light-sensitive area of the detector 220.

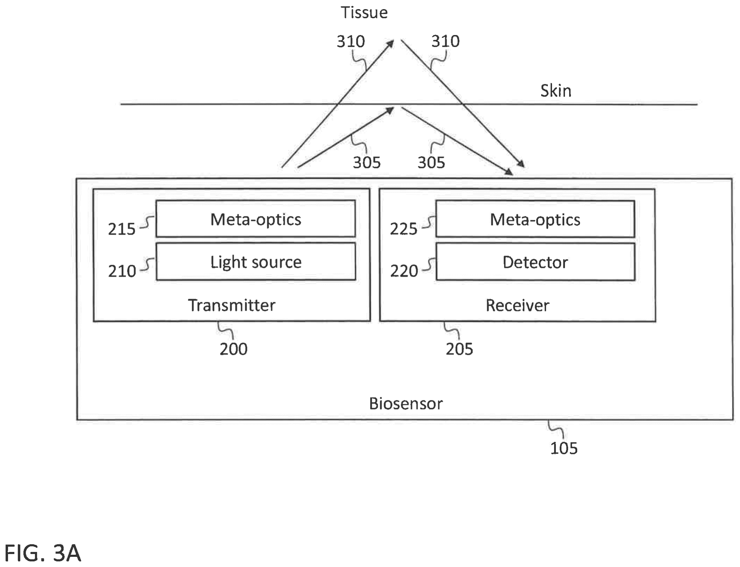

In some embodiments, the transmitter 200 emits polarized light, or the receiver 205 receives light in a polarization-dependent manner. FIG. 3A illustrates two light paths that light may take from the transmitter 200 to the receiver 205. A first path 305 exits the transmitter 200, reflects from the exterior surface of the skin of the subject, and is received by the receiver 205. A second path 310 exits the transmitter 200, passes through the exterior surface of the skin, interacts with the tissue of the subject, exits from the tissue of the subject, and is received by the receiver 205. The second path 310 is simplified in FIG. 3A for ease of illustration; each ray of light that propagates within the tissue of the subject and returns to the receiver 205 may scatter multiple times before returning to the receiver 205. A substantial portion of the light emitted by the transmitter 200 may follow a path that does not end at the receiver; as such, a substantial portion of the light emitted by the transmitter 200 may be lost.

Light that has propagated along the second path 310 may carry more useful information than the light that has propagated along the first path 305, because the light that has propagated along the second path 310 may have interacted more extensively with the tissue. As such, it may be advantageous to reduce the contribution, made by light that has propagated along the first path 305, to the light received by the detector 220.

This may be accomplished by controlling the polarization of the transmitted light and the polarization of the light that reaches the detector 220. The extent to which light reflects from the exterior surface of the skin of the subject may depend on the polarization of the light. For example, horizontally polarized light may reflect to a greater extent from the exterior surface of the skin of the subject than vertically polarized light. As such, a polarizer in the receiver 205 that blocks horizontally polarized light may preferentially attenuate the light that has propagated along the first path 305, thereby improving the performance of the biosensor 105. Such a polarizer may be implemented in the second set of meta-optics 225, e.g., by including in the second set of meta-optics 225 an optical element with a metasurface (which may be referred to as a second metasurface) that functions as a polarizer.

In some embodiments, the second metasurface is a polarizer that is oriented so as to reject light transmitted by the infrared transmitter and received at the receiver via a single specular reflection. In such an embodiment, the light that has propagated along the first path 305 may be attenuated, regardless of the polarization of the light produced by the transmitter 200. Light that has propagated along the second path 310 may be unpolarized, or only partially polarized, as a result of the interactions between the light that has propagated along the second path 310 and the tissue of the subject, and, as such, it may be less strongly attenuated by the second metasurface than the light that has propagated along the first path 305.

In some embodiments, both measures for reducing the contribution from the light that has propagated along the first path 305 are employed, e.g., (i) the second set of meta-optics 225 includes a second metasurface, which operates as a polarizer which blocks horizontally polarized light and (ii) the first set of meta-optics 215 includes a polarizer that causes the transmitter to emit light that is polarized such that the second metasurface rejects light transmitted by the infrared transmitter and received at the receiver via a single specular reflection.

As used herein, "horizontally polarized" light is light the electric field of which is parallel to the skin of the subject, and "vertically polarized" light is light having an electric field perpendicular to that of horizontally polarized light.

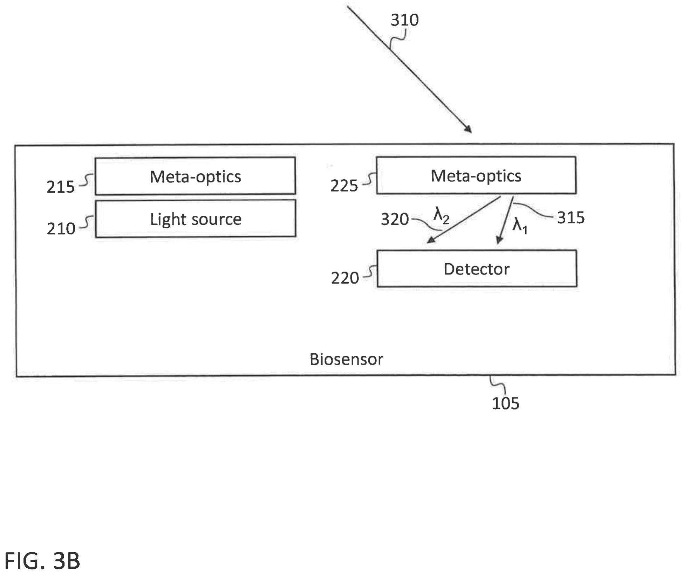

In some embodiments, the second set of meta-optics 225 disperses the light received by the receiver 205. Such an embodiment is illustrated in FIG. 3B. Light that has propagated along the second path 310 is received by the second set of meta-optics 225 and dispersed, so that a first portion of the light 315 (e.g., a portion having a wavelength λ₁) falls on a first portion of the detector 220, and a second portion of the light 320 (e.g., a portion having a wavelength λ₂) falls on a second portion of the detector 220. In such an embodiment, if the detector is an array detector (e.g., a focal plane array) it may be possible for the biosensor 105 to measure the spectrum of the received light, and, as such, infer characteristics of the tissue of the subject (such as the concentration of a biomolecule) that may affect the spectral response of the tissue of the subject.

To be capable of functioning in the thermal infrared region of the electromagnetic spectrum, the materials of the optical elements of the first set of meta-optics 215 and of the second set of meta-optics 225 may be constructed using materials that are transparent in the thermal infrared region of the electromagnetic spectrum. Fused silica may be opaque in the thermal infrared region of the electromagnetic spectrum, and, as such, may not be suitable. In some embodiments, the optical elements of the first set of meta-optics 215 and of the second set of meta-optics 225 may be constructed of a substrate composed of calcium fluoride, silicon, germanium, or a II-VI chalcogenide. The metasurface may be composed of the same material as the substrate, or of a material selected (i) from among a plurality of organic polymers, e.g., from the group consisting of SU8, polyethylene, cyclic olefin copolymer (COC), polydimethylsiloxane (PDMS), polyethylene (PE, HDPE), polycarbonate (PC), polyethylene terephthalate (PET), and polymethyl methacrylate (PMMA), or (ii) from among a plurality of inorganic dielectric molecules, e.g., from the group consisting of silicon nitride (Si₃N₄), aluminum nitride (AIN), titanium dioxide (TiO₂), gallium nitride (GaN), and zinc selenide (ZnSe). These materials may exhibit low absorption loss, high refractive index tunability, and compatibility with nanofabrication techniques for infrared wavelength operation.

Each optical element may include a substrate and a metasurface on one surface of the substrate, or two metasurfaces, one on each side of the substrate. In such an embodiment, the metasurfaces on the two sides of the substrate may be different and the optical element may have more than one function as a result. For example, the optical element may have (i) a metasurface on one surface of the substrate that functions as a lens, and (ii) a metasurface on the other surface of the substrate that functions as a polarizer. In such an embodiment, the optical element may function as a lens and it may further function as a polarizer. In some embodiments, a metasurface is constructed to perform multiple functions. For example, a metasurface on an optical element may function as a lens and it may further function as a polarizer.

Each metasurface may be fabricated using deep ultraviolet lithography, or using imprinting (e.g., using a metal stamp or a silicon stamp). In some embodiments, an imprinted polymer layer is used as the mask for a lithography operation. In some embodiments the stamp is transparent to ultraviolet light, and the stamp is used to imprint a polymer to form the metasurface, and then left in place while the polymer is cured using ultraviolet light which illuminates the polymer through the stamp. Metasurfaces may also be fabricated using other top-down methods such as electron-beam lithography, and ion-beam milling - which may be more expensive but may nonetheless be used for precise structures. In some embodiments, a metasurface may be fabricated using a bottom-up method (which may use small particles, assembled into nanostructures), such as colloidal nanoparticle self-assembly, deoxyribonucleic acid (DNA) origami, or chemical synthesis (to grow particles into desired shapes). In some embodiments, a metasurface may be fabricated using two-photon polymerization (which may be used to perform 3D printing with high resolution). Such fabrication methods may be combined with additional fabrication techniques which may deposit or etch thin layers of metals or dielectric materials. Such a method may be used to add layers of material or to remove existing materials to form a desired shape and pattern. In some embodiments, a thin layer deposition method such as sputtering, thermal evaporation, e-beam evaporation, atomic layer deposition, or chemical vapor deposition is used. In some embodiments, an etching method such as reactive ion etching, dry etching (using gases), or wet etching (using liquids) may be used.

FIG. 4 is a schematic view of the electronic device according to some embodiments. Referring to FIG. 4, various electronic devices into which the biosensor 105 may be incorporated may include a smartphone 405, a tablet computer 410, a laptop computer 415, a wearable electronic device including a display module such as a smart glasses 425, a head mounted display 430, and a smart watch 420, and a vehicle electronic device including a display module such as an instrument panel, a center fascia, a center information display (CID) located on a dashboard, and a room mirror display of a vehicle.

In some embodiments, a system includes: a biosensor, including: an infrared transmitter; and an infrared receiver, the infrared transmitter including a light source and a first metasurface, the infrared transmitter being configured to transmit light, from the light source, through the first metasurface, the infrared receiver including a detector and a second metasurface, the detector being configured to receive light which enters the infrared receiver through the second metasurface. For example, as discussed in further detail above in the context of FIG. 2A, the biosensor 105 may include a transmitter 200 including a light source 210 and a first set of meta-optics 215. The first set of meta-optics 215 may include a first metasurface. The biosensor 105 may further include a receiver 205, which may include a second set of meta-optics 225 including a second metasurface.

In some embodiments, the first metasurface functions as a polarizer, and in some embodiments, the second metasurface functions as a polarizer. In some embodiments, the first metasurface is oriented so that the infrared transmitter emits vertically polarized light. In some embodiments, the second metasurface is oriented so as to reject light transmitted by the infrared transmitter and received at the receiver via a single specular reflection. In some embodiments, the first metasurface is oriented so that the infrared transmitter emits vertically polarized light. Such characteristics may reduce the amount of probe light, directly reflected from the exterior surface of the skin of the user, that reaches the detector 220. In some embodiments, the first metasurface further functions as a lens, e.g., for collimating or focusing the light from the light source 210.

Signal detection and processing may include signal detection by the detector 220, and processing by one or more of the processing elements of the system (e.g., by the microcontroller unit 235 or the digital signal processor 240). Such processing may include, for example, inferring or estimating one or more characteristics of the tissue of the subject (as discussed in further detail elsewhere herein) based on the extent to which light is transmitted, from the transmitter 200, and, after interacting with the tissues of the subject, to the receiver 205. The biosensor 105 may be configured to measure a spectral transmission fraction. The transmission fraction may be the fraction of the light transmitted by the transmitter 200 that is received, after interacting with the tissue of the subject, by the receiver 205. The spectral transmission fraction may be the transmission fraction per unit wavelength as a function of wavelength, and it may be represented, e.g., in the microcontroller unit 235 or in the digital signal processor 240, as a one-dimensional array, each element of the array representing the spectral transmission fraction at a different respective wavelength. The measuring of the spectral transmission fraction may be accomplished, for example, by causing the transmitter 200 to emit at one wavelength (or in a narrow range of wavelengths centered on one wavelength) at a time, or by arranging for different wavelengths to be separately detected in the receiver, e.g., by (i) using, as discussed above, a pixelated metasurface with pixels aligned with light source pixels or aligned with detector pixels, or by (ii) using, in the receiver 205, a pixelated detector 220 and a metasurface that disperses light so that each of a plurality of different wavelengths falls on (and is independently detected by) a different respective pixel of the detector 220.

The signal to noise ratio (SNR) of the biosensor 105 may depend on a number of factors, including the radiant intensity of the transmitter 200 along a direction in which the transmission fraction is greatest, the sensitivity of the detector 220, and the noise equivalent power of the detector. The signal to noise ratio (SNR) of the biosensor 105 may further depend on the power spectral density, as measured at the output of the detector 220, of external noise sources such as the signal caused by light reflected from the surface of the skin of the subject and such as ambient light reaching the detector 220. In some embodiments, the output of the transmitter 200 is modulated (e.g., at a frequency between 1 Hz and 10 kHz) and the electrical signal at the output of the detector 220 is demodulated with the same frequency (e.g., demodulated by the microcontroller unit 235, after conversion of the analog output of the detector 220 to a digital signal). In such an embodiment, the effect of low-frequency noise may be reduced.

The measured spectral transmission fraction may be used for biosensing, e.g., to provide various kinds of information about the tissue with which the light has interacted. For example, the absorption spectra of various molecules of the tissue may have absorption lines the width of which may depend (e.g., as a result of collisional broadening or doppler broadening) on the temperature of the tissue; as such, a measurement of such line widths may be used to infer the core body temperature of the subject. As another example, the spectrum or radiant intensity of blackbody radiation emitted from the tissues (e.g., during time intervals when the transmitter 200 is not emitting light) may be used to infer the core body temperature of the subject.

Other examples include (i) calorimetric biosensing, in which heat changes (e.g., changes in enthalpy) from biochemical reactions (e.g. enzyme activity, binding events) may be measured and the rates of such reactions may be inferred, (ii) the use of the receiver 205 to monitor temperature changes or patterns on the skin or in the tissue of the subject to detect inflammation, infection, or blood flow abnormalities, or to perform vascular diagnostics, (iii) cancer cell detection by heating the skin and detecting a cancer cell specific heat map or infrared map, and (iv) the measurement or detection of binding events on a surface within the tissue (such as antibodies capturing antigens), which may change the local heat transfer resistance and which may therefore be captured by the biosensor 105, enabling label-free biosensing.

In some embodiments, the second metasurface further functions as a lens, for focusing the received light on the detector 220. In some embodiments, the second metasurface further functions as a disperser, which, as discussed above in the context of FIG. 3B, may enable the biosensor 105 to measure the spectral response of the tissue of the subject. In some embodiments, an apparatus includes: an electronic device, including the biosensor 105.

As used herein, "a portion of" something means "at least some of" the thing, and as such may mean less than all of, or all of, the thing. As such, "a portion of" a thing includes the entire thing as a special case, i.e., the entire thing is an example of a portion of the thing. As used herein, when a second quantity is "within Y" of a first quantity X, it means that the second quantity is at least X-Y and the second quantity is at most X+Y. As used herein, when a second number is "within Y%" of a first number, it means that the second number is at least (1-Y/100) times the first number and the second number is at most (1+Y/100) times the first number. As used herein, the term "or" should be interpreted as "and/or", such that, for example, "A or B" means any one of "A" or "B" or "A and B".

Each of the terms "processing circuit" and "means for processing" is used herein to mean any combination of hardware, firmware, and software, employed to process data or digital signals. Processing circuit hardware may include, for example, application specific integrated circuits (ASICs), general purpose or special purpose central processing units (CPUs), digital signal processors (DSPs), graphics processing units (GPUs), and programmable logic devices such as field programmable gate arrays (FPGAs). In a processing circuit, as used herein, each function is performed either by hardware configured, i.e., hard-wired, to perform that function, or by more general-purpose hardware, such as a CPU, configured to execute instructions stored in a non-transitory storage medium. A processing circuit may be fabricated on a single printed circuit board (PCB) or distributed over several interconnected PCBs. A processing circuit may contain other processing circuits; for example, a processing circuit may include two processing circuits, an FPGA and a CPU, interconnected on a PCB.

As used herein, when a method (e.g., an adjustment) or a first quantity (e.g., a first variable) is referred to as being "based on" a second quantity (e.g., a second variable) it means that the second quantity is an input to the method or influences the first quantity, e.g., the second quantity may be an input (e.g., the only input, or one of several inputs) to a function that calculates the first quantity, or the first quantity may be equal to the second quantity, or the first quantity may be the same as (e.g., stored at the same location or locations in memory as) the second quantity.

It will be understood that, although the terms "first", "second", "third", etc., may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed herein could be termed a second element, component, region, layer or section.

Spatially relative terms, such as "beneath", "below", "lower", "under", "above", "upper" and the like, may be used herein for ease of description to describe one element or feature's relationship to another element(s) or feature(s) as illustrated in the figures. It will be understood that such spatially relative terms are intended to encompass different orientations of the device in use or in operation, in addition to the orientation depicted in the figures. For example, if the device in the figures is turned over, elements described as "below" or "beneath" or "under" other elements or features would then be oriented "above" the other elements or features. Thus, the example terms "below" and "under" can encompass both an orientation of above and below. The device may be otherwise oriented (e.g., rotated 90 degrees or at other orientations) and the spatially relative descriptors used herein should be interpreted accordingly. In addition, it will also be understood that when a layer is referred to as being "between" two layers, it can be the only layer between the two layers, or one or more intervening layers may also be present.

The terminology used herein is for the purpose of describing particular embodiments only and is not intended to be limiting of the inventive concept. As used herein, the terms "substantially," "about," and similar terms are used as terms of approximation and not as terms of degree, and are intended to account for the inherent deviations in measured or calculated values that would be recognized by those of ordinary skill in the art.

As used herein, the term "major component" refers to a component that is present in a composition, polymer, or product in an amount greater than an amount of any other single component in the composition or product. In contrast, the term "primary component" refers to a component that makes up at least 50% by weight or more of the composition, polymer, or product. As used herein, the term "major portion", when applied to a plurality of items, means at least half of the items. As used herein, any structure or layer that is described as being "made of" or "composed of" a substance should be understood (i) in some embodiments, to contain that substance as the primary component or (ii) in some embodiments, to contain that substance as the major component.

It will be further understood that the terms "comprises" and/or "comprising", when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Expressions such as "at least one of," when preceding a list of elements, modify the entire list of elements and do not modify the individual elements of the list. Further, the use of "may" when describing embodiments of the inventive concept refers to "one or more embodiments of the present disclosure". Also, the term "exemplary" is intended to refer to an example or illustration. As used herein, the terms "use," "using," and "used" may be considered synonymous with the terms "utilize," "utilizing," and "utilized," respectively.

It will be understood that when an element or layer is referred to as being "on", "connected to", "coupled to", or "adjacent to" another element or layer, it may be directly on, connected to, coupled to, or adjacent to the other element or layer, or one or more intervening elements or layers may be present. In contrast, when an element or layer is referred to as being "directly on", "directly connected to", "directly coupled to", or "immediately adjacent to" another element or layer, there are no intervening elements or layers present.

Any numerical range recited herein is intended to include all sub-ranges of the same numerical precision subsumed within the recited range. For example, a range of "1.0 to 10.0" or "between 1.0 and 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Similarly, a range described as "within 35% of 10" is intended to include all subranges between (and including) the recited minimum value of 6.5 (i.e., (1 - 35/100) times 10) and the recited maximum value of 13.5 (i.e., (1 + 35/100) times 10), that is, having a minimum value equal to or greater than 6.5 and a maximum value equal to or less than 13.5, such as, for example, 7.4 to 10.6. Any maximum numerical limitation recited herein is intended to include all lower numerical limitations subsumed therein and any minimum numerical limitation recited in this specification is intended to include all higher numerical limitations subsumed therein.

## Claims

1. A biosensor (105) comprising:
an infrared transmitter (200); and
an infrared receiver (205),
the infrared transmitter (200) comprising a light source (210) and a first metasurface (215), the infrared transmitter (200) being configured to transmit light, from the light source (210), through the first metasurface (215),
the infrared receiver (205) comprising a detector (220) and a second metasurface (225), the detector (220) being configured to receive light which enters the infrared receiver (205) through the second metasurface (225).

2. The biosensor (105) of claim 1, wherein the first metasurface functions as a polarizer.

3. The biosensor (105) of claim 1 or 2, wherein the first metasurface (215) further functions as a lens.

4. The biosensor (105) of any one of claims 1 to 3, wherein the second metasurface functions as a polarizer.

5. The biosensor (105) of any one of claims 1 to 4, wherein the first metasurface (215) is oriented so that the infrared transmitter (200) emits vertically polarized light.

6. The biosensor (105) of any one of claims 1 to 5, wherein the second metasurface (225) is oriented so as to reject light transmitted by the infrared transmitter (200) and received at the receiver (205) via a single specular reflection.

7. The biosensor (105) of any one of claims 1 to 6, wherein the first metasurface (215) is oriented so that the infrared transmitter (200) emits vertically polarized light.

8. The biosensor (105) of any one of claims 1 to 7, wherein the second metasurface (225) further functions as a lens.

9. The biosensor (105) of any one of claims 1 to 8, wherein the second metasurface (225) further functions as a disperser.

10. The biosensor (105) of any one of claims 1 to 9, wherein:
the light source (210) comprises a first pixel and a second pixel; and
the first metasurface (215) comprises:
a first pixel aligned with the first pixel of the light source (210), and
a second pixel aligned with the second pixel of the light source (210).

11. The biosensor (105) of any one of claims 1 to 10, wherein the biosensor (105) further comprises a temperature sensor (255) configured to measure ambient temperature.

12. The biosensor (105) of any one of claims 1 to **11,** wherein the light source (210) comprises an element selected from the group consisting of broadband thermal sources, quantum cascade lasers, optical parametric oscillators, and super continuum lasers.

13. The biosensor (105) of any one of claims 1 to 12, wherein the detector (220) comprises an element selected from the group consisting of thermopile detectors (220), pyroelectric detectors (220), bolometers, photonic detectors (220), and focal plane arrays.

14. A System-on-a-Chip, SoC (230), which is a silicon integrated circuit and includes the biosensor 105 of any one of claims 1 to 13.
